# EUROPÄISCHE PATENTSCHRIFT

(11) **EP 2 501 818 B1**
(45) Veröffentlichungstag und Bekanntmachung des Hinweises auf die Patenterteilung: **26.12.2018**
(21) Anmeldenummer: 10721517.0
(22) Anmeldetag: 01.06.2010
(51) Int. Cl.: C12P 7/06, C12P 5/02

(54) **ENERGETISCH OPTIMIERTES VERFAHREN ZUM BETREIBEN EINER BIOETHANOLGEWINNUNGSANLAGE**
ENERGY-OPTIMIZED METHOD FOR OPERATING A BIOETHANOL PRODUCTION PLANT
PROCÉDÉ OPTIMISÉ AU PLAN ÉNERGÉTIQUE POUR L'EXPLOITATION D'UNE INSTALLATION DE PRODUCTION DE BIOÉTHANOL

(30) Priorität: 02.06.2009 DE 102009023573; 27.01.2010 DE 102010005818
(43) Veröffentlichungstag der Anmeldung: 26.09.2012
(73) Patentinhaber: Verbio Vereinigte Bioenergie AG, 04109 Leipzig (DE)
(72) Erfinder: POLLERT, Georg, 13465 Berlin (DE); LÜDTKE, Oliver, 04416 Markkleeberg (DE); FICHTER, Enrico, 04315 Leipzig (DE); BETTIEN, Klaus-Dieter, 16303 Schwedt/Oder (DE)
(74) Vertreter: Kruspig, Volkmar
(86) Internationale Anmeldenummer: PCT/EP2010/057657
(87) Internationale Veröffentlichungsnummer: WO 2010/139699

(56) Entgegenhaltungen:
- EP-A1- 2 060 635
- EP-A2- 2 072 615
- WO-A1-01/60752
- WO-A1-2008/003692
- WO-A1-2010/100224
- WO-A2-2010/125517
- DE-U1-202007 017 698
- US-A1- 2005 153 410
- US-A1- 2007 190 626
- Matthew T. Agler ET AL: "Thermophilic Anaerobic Digestion to Increase the Net Energy Balance of Corn Grain Ethanol", Environmental Science & Technology, vol. 42, no. 17, 1 September 2008 (2008-09-01), pages 6723-6729, XP055311010, US ISSN: 0013-936X, DOI: 10.1021/es800671a

## Beschreibung

Die Erfindung betrifft ein energetisch optimiertes Verfahren zum Betreiben einer Bioethanolgewinnungsanlage, wobei die anfallende Schlempe in eine Biogasanlage eingeleitet wird, um enthaltene organische Substanzen in Biogas umzuwandeln, zur Durchführung eines kombinierten Betriebs der Bioethanol- und Biogaserzeugung gemäß Oberbegriff des Patentanspruchs 1.

Aus der DE 10 2007 015 623 A1 ist ein Verfahren zur energetischen Nutzung von landwirtschaftlich erzeugten Rohstoffen vorbekannt.

Gemäß dem dortigen Verfahren soll die Aufgabe gelöst werden, ein Heizkraftwerk so weiterzubilden, indem der Dampfturbine eine verfahrenstechnische Anwendung nachgeordnet ist, in der ein Teil der im Prozessdampf enthaltenen Energie verbraucht wird und für den Restdampf eine Bioethanolanlage zur Erzeugung von Bioethanol nachgeschaltet ist, für deren energetische Prozesse ein Teil der im Restdampf enthaltenen Energie nutzbar ist.

Hierfür wird die in dem aus der Turbine ausströmenden Prozessdampf enthaltene Energie teilweise in der nachgeschalteten Anwendung genutzt, wobei die in dem Restdampf aus der verfahrenstechnischen Anwendung enthaltene Energie in der nachgeschalteten Bioethanolanlage zur Herstellung von Bioethanol verwendet wird.

Bei einer Ausführungsform nach DE 10 2007 015 623 A1 wird darauf hingewiesen, dass bei der Biogaserzeugung auch geeignete biologische Reststoffe verwertet werden können, wie insbesondere die sogenannte Schlempe aus der Bioethanolerzeugung. Diese Schlempe weist einen hohen Anteil organischer Stoffe auf. Daher ist selbige sehr gut für die Biogaserzeugung geeignet. Durch die Verwertung der Schlempe zur Biogaserzeugung reduziert sich die ansonsten notwendige Entsorgungslogistik. Es soll also durch die Aufarbeitung der Schlempe zu Biogas die Energieeffizienz einer entsprechenden Anlage und die Ausnutzung des theoretisch nutzbaren pflanzlichen Potentials insgesamt gesteigert werden.

Bei dem Verfahren zur Herstellung von Ethanol aus Getreide gemäß DE 10 2007 033 988 A1 ist u.a. dargelegt, dass bei dem Gesamtverfahren zur Bioethanolherstellung sehr große Mengen an Wasser benötigt werden. Für die Einmaischung werden hier pro Tonne des verwendeten Getreides bis zu 3,5 m³ Frischwasser notwendig. Um den Wasserverbrauch zu senken, wird nach der DE 10 2007 033 988 A1 vorgeschlagen, eine auf bestimmte Weise gereinigte Stärke als Fermentationsedukt zu verwenden.

Letztendlich legt die DE 10 2006 040 567 A1 in dem dortigen Verfahren zur Herstellung von Bioethanol aus Biomasse vor allem Getreide und insbesondere Roggen dar, dass bei der Erzeugung von Ethanol aus Roggen recht hohe Konversionskosten entstehen. Diese höheren Kosten resultieren aus der Anwendung viskositätsenkender Enzyme, um der Verschleimung im Konversionsprozess zu begegnen und dem höheren Dampfbedarf bei der DDGS (Destillers' Dried Grains with Solubles) Trocknung. Eine Schlemperückführung ist nach DE 10 2006 040 567 A1 nur nach Maßgabe der Einmaischkonzentration möglich und schwankt damit in Abhängigkeit von der Viskosität.

Zum Stand der Technik sei noch auf das energieautarke Verfahren zur Herstellung von Bioethanol gemäß DE 10 2007 001 614 A1 verwiesen. Gemäß dem dortigen Verfahren wird die in einer Phasentrennung abgetrennte Dicksauermaische zum Zweck der Gewinnung von Biomethan für die Erzeugung von Prozessenergie in einem Blockheizkraftwerk einer parallel betriebenen Methanisierungsstufe zugeführt. Bei einer weiteren Variante des dortigen Verfahren wird als Prozesswasser das Kondensat aus destilliertem Schlempewasser eingesetzt. Darüber hinaus soll sich das als Schlempewasser anfallende Sumpfprodukt auch wenigstens teilweise zur Gewinnung von Prozesswasser verwenden lassen, indem zur Gewinnung von salzarmem Prozesswasser und salzreichem Konzentrat aus dem Schlempewasser eine Vakuum-Umlaufverdampfungsanlage, eine Mikrofiltrations-, Ultrafiltrations-, Nanofiltrationssowie Umkehrosmose- und/oder Membrantechniken eingesetzt werden.

Durch die derzeitige Förderung ist die Erzeugung von Ethanol als Ersatzstoff für Autokraftstoff zu einem bedeutenden Faktor in der Landwirtschaft geworden. Das Motiv der staatlichen Förderung liegt hauptsächlich in der Verringerung des CO₂-Ausstoßes in die Atmosphäre im Transportsektor.

In Ethanolanlagen muss das Produkt unter Einsatz von großen Mengen Wasser erzeugt werden, so dass entsprechend große Mengen dieses Wassers am Ende des Prozesses zusammen mit dem im Vergärungsprozess nicht verwertbaren Inhaltsstoffen als Schlempe anfällt. Obiges ist ein grundlegendes Problem, da dieses Wasser in irgendeiner Form entsorgt werden muss. Bei konventionellen Verfahren wird die Schlempe zur Gewinnung von Tierfutter (DDGS) eingedampft und getrocknet. Auch hier werden große Mengen Energie verbraucht, wodurch der CO₂-Einspareffekt konterkariert wird, da die Gesamtbilanz der CO₂-Einsparung damit einer drastischen Verschlechterung unterliegt.

Alternativ kann die Schlempe als NPK-Dünger auf die Felder in der Umgebung der Produktionsanlage ausgebracht werden. Dies hat aber auch verschiedene gravierende Nachteile. Zum einen ist das Verfahren der Düngerausbringung wenig wirtschaftlich, da die Landwirtschaft den Wert des Düngers kaum vergütet und die organischen Bestandteile der Wertschöpfung verloren gehen.

Zum anderen ist die Ausbringung auch mit einem hohen energetischen Aufwand verbunden, da sehr große Wassermengen über zum Teil erhebliche Distanzen transportiert werden müssen. Außerdem ist das Ausbringen zeitlich durch eine Kernsperrfrist im Winter beschränkt.

Aus Obigem ist unabhängig von der Art und Weise, wie die Schlempe verwertet oder entsorgt wird, die große Wasermenge, die im Prozess der alkoholischen Gärung notwendig ist, ein grundlegendes Problmen aller Ethanolgewinnungsanlagen. Eine Möglichkeit, diese Wasserströme zu verringern, ist das Recycling. Beschränkt wird diese Möglichkeit durch die Anreicherung von Inhaltsstoffen, die ab einem bestimmten Anreicherungsgrad einen normalen Betrieb nicht mehr zulässt; sei es durch eine nicht mehr beherrschbare Viskosität oder durch Erhöhung der Konzentration von Stoffen bis hin zur Giftigkeit für die Hefe.
Durch bestimmte Maßnahmen können diese schädlichen Einflüsse zum Teil verringert oder ausgeschaltet werden. So kann der Recyclinggrad allein durch den Einsatz von Dekantern, in denen gröbere Inhaltsstoffe abgeschieden werden, erheblich erhöht werden, da dadurch die Viskosität der Schlempe stark reduziert wird. Letztendlich ist aber das Zurückführen der Schlempe durch ihre hohen organischen, nicht abtrennbaren Bestandteile beschränkt.

Wie eingangs dargelegt, können diese Nachteile teilweise dadurch vermieden werden, indem die Schlempe in einer Biogasanlage anaerob fermentiert wird. Bei diesem Prozess werden die organischen Bestandteile der Schlempe weitgehend zu Methan und CO₂ umgesetzt. Die Verwertung von Schlempe aus dem Ethanolgewinnungsprozess als Monosubstrat in Biogasanlagen ist bisher allerdings großtechnisch nicht realisiert worden. Grundsätzlich ist damit aber das Problem der großen Wassermengen, die benötigt werden, nicht gelöst, da dann die Notwendigkeit besteht, das Wasser als Ablauf der Biogasanlage zu entsorgen.

Aus dem Vorgenannten ist es daher Aufgabe der Erfindung, ein weiterentwickeltes energetisch optimiertes Verfahren zum Betreiben einer Bioethanolgewinnungsanlage anzugeben, wobei die anfallende Schlempe in eine Biogasanlage eingeleitet wird, um enthaltene organische Substanzen in Biogas umzuwandeln, und zwar zum Zweck der Durchführung eines kombinierten Betriebs der Bioethanol- und Biogaserzeugung mit dem Ziel, eine Reduzierung der benötigten Frischwassermengen zu erreichen.

Die Lösung der Aufgabe der Erfindung erfolgt durch die Lehre gemäß Patentanspruch 1, wobei die Unteransprüche mindestens zweckmäßige Ausgestaltungen und Weiterbildungen umfassen.

Bei dem erfindungsgemäßen Verfahren wird demnach eine direkte Rückführung eines Teils des Ablaufs der Biogasanlage zum Zweck des Einmaischens in den Fermenter der Bioethanolgewinnungsanlage vorgenommen derart, dass der Frischwassereinsatz bezogen auf den Gesamtprozess einer wesentlichen Reduzierung unterliegt.

Der Ablauf der Biogasanlage kann bei einer Ausführungsform teilweise einem Feststoff-Abtrennschritt unterzogen werden.

Der so behandelte, gereinigte Ablauf wird dann der Bioethanolgewinnungsanlage zugeführt.

Der Abtrennschritt kann eine Filtration und/oder eine Dekantierung umfassen.

Ebenfalls liegt es im Sinn der Erfindung, aus dem Ablauf der Biogasanlage einen Teilstrom auszuschleusen, mittels eines Membranverfahrens diesen Teilstrom zu reinigen und das erhaltene Permeat ganz oder teilweise zum Anmaischen in die Bioethanolgewinnungsanlage einzubringen.

Ebenso besteht die Möglichkeit, aus dem Ablauf der Biogasanlage einen Teilstrom auszuschleusen, einzudampfen und damit aufzukonzentrieren. Das Kondensat kann dann mindestens teilweise zum Anmaischen in die Bioethanolgewinnungsanlage eingebracht werden.

Ferner besteht erfindungsgemäß die Möglichkeit, in der Biogasanlage gebildetes Ammoniak durch Stripping bis auf eine für den Gesamtprozess unschädliche Konzentration zu entfernen.

Die vorliegende Anmeldung offenbart ferner die folgenden, nicht beanspruchten, Verfahrensmerkmale.

Der im Rücklauf enthaltene Schwefelwasserstoff kann entfernt werden. Dieses Entfernen kann z.B. durch Entgasen und/oder Fällen erfolgen.

Es kann dafür gesorgt werden, im Gesamtprozess an geeigneter Stelle Ammoniak und/oder das Ammonium entsprechender Salze zu entfernen, um eine optimale Prozessführung zu gewährleisten.

Die Erfindung soll nachstehend anhand eines Ausführungsbeispiels näher erläutert werden.

Es wurde überraschenderweise festgestellt, dass die direkte Rückführung des Ablaufs der Biogasanlage zum Einmaischen des Getreides ohne wesentliche Beeinträchtigung des Fermentationsprozesses möglich ist.

Durch diese Maßnahme kann der Einsatz von Frischwasser auf einen Bruchteil des üblicherweise notwendigen Bedarfs gesenkt oder ganz vermieden werden.

Bei einer Betrachtung der Bilanz des Gesamtsystems wird vorgeschlagen, an einer oder mehreren Stellen einen Auslass zu schaffen, da aus dem ganzen Getreidekorn in der Ethanolgewinnungsanlage zwar die Stärke und in der Biogasanlage organische Inhaltsstoffe, wie z.B. Eiweiß und die der Hefe nicht zugänglichen Pentosane abgebaut werden, hingegen Zellulose und ähnliche organische Substanzen sowie alle anorganischen Inhaltsstoffe mit Ausnahme des Schwefels, der zu Schwefelwasserstoff reduziert wird und das System über das Biogas verlässt, in der flüssigen Phase verbleiben.

Es wird daher erfindungsgemäß sichergestellt, dass eine Anreicherung der weder in der ethanolischen Gärung noch in der Biogasanlage abbaubaren oder der sich bildenden Stoffe, wie z.B. Ammoniak, auf eine für den Gesamtprozess schädliche Konzentration unterbleibt. Es werden daher diese schädlichen Stoffe aus dem Prozesslauf entfernt.

Das notwendige Ausschleusen kann prinzipiell an jeder, aber auch an mehreren Prozessstellen im System vorgenommen werden.

So werden z.B. beim Abtrennen gröberer Substanzen aus der Dickschlempe im Dekanter erhebliche Mengen gelöster Stoffe, wie Mineralsalze, ausgeschleust, da der abgetrennte Treber ca. 65% Wasser enthält. Ein wichtiger Schritt gemäß Ausführungsbeispiel ist die Abtrennung des Ammoniaks durch Strippung in einem entsprechenden Verfahrensschritt.

Weitere Möglichkeiten bestehen in der Ausschleusung eines Teilstroms der umlaufenden Flüssigkeit und deren weitere Behandlung, wie z.B. durch Eindampfung.

Insgesamt wird der energetische und apparative Aufwand zur Entsorgung ansonsten anfallender großer anfallender Wassermengen, die der Ethanolprozess nach sich zieht, auf einen Bruchteil bisheriger Aufwendungen und Kosten gesenkt.

## Patentansprüche

1. Verfahren zur Durchführung eines kombinierten Betriebs einer Bioethanolgewinnungsanlage und einer Biogasanlage, **dadurch gekennzeichnet:**
- **dass** die in der Bioethanolgewinnungsanlage anfallende Schlempe in eine Biogasanlage eingeleitet wird, um enthaltene organische Substanzen in Biogas umzuwandeln, und
- **dass** eine direkte Rückführung eines Teils des Ablaufs der Biogasanlage zum Zweck des Einmaischens in den Fermenter der Bioethanolgewinnungsanlage erfolgt.

2. Verfahren nach Anspruch 1, **dadurch gekennzeichnet, dass** der Ablauf der Biogasanlage vor der direkten Rückführung teilweise einem Feststoff-Abtrennschritt unterzogen wird.

3. Verfahren nach Anspruch 2, **dadurch gekennzeichnet, dass** der Abtrennschritt eine Filtration und/oder Dekantierung umfasst.

4. Verfahren nach einem der vorangegangenen Ansprüche, **dadurch gekennzeichnet, dass** aus dem Ablauf der Biogasanlage ein Teilstrom ausgeschleust wird, welcher mittels eines Membranverfahrens gereinigt wird und weiterhin das erhaltene Permeat ganz oder teilweise zum Anmaischen in die Bioethanolgewinnungsanlage gelangt.

5. Verfahren nach einem der vorangegangenen Ansprüche, **dadurch gekennzeichnet, dass** aus dem Ablauf der Biogasanlage ein Teilstrom ausgeschleust, eingedampft und damit aufkonzentriert wird sowie das Kondensat mindestens teilweise zum Anmaischen in die Bioethanolgewinnungsanlage gelangt.

6. Verfahren nach einem der vorangegangenen Ansprüche, **dadurch gekennzeichnet, dass** in der Biogasanlage gebildetes Ammoniak durch Strippung bis auf eine für den Gesamtprozess unschädliche Konzentration entfernt wird.

## Claims

1. Method for carrying out a combined operation of a bioethanol production plant and a biogas plant, **characterized in that**:
- the vinasse produced in the bioethanol production plant is fed into a biogas plant in order to convert the organic substances contained therein into biogas, and
- part of the effluent of the biogas plant is directly re-fed for the purpose of mashing in the fermenter of the bioethanol production plant.

2. Method according to claim 1, **characterized in that** the effluent of the biogas plant is partially subjected to a solids separation step prior to direct re-feeding.

3. Method according to claim 2, **characterized in that** the separation step comprises filtration and/or decantation.

4. Method according to one of the preceding claims, **characterized in that** a partial stream is discharged from the effluent of the biogas plant, which partial stream is cleaned by means of a membrane process and furthermore the obtained permeate reaches the bioethanol production plant in full or in part for initial mashing.

5. Method according to one of the preceding claims, **characterized in that** a partial stream is discharged from the effluent of the biogas plant, evaporated and thus concentrated, and the condensate is at least partially conveyed to the bioethanol production plant for initial mashing.

6. Method according to one of the preceding claims, **characterized in that** ammonia formed in the biogas plant is removed by stripping to a concentration which is harmless to the overall process.

## Revendications

1. Procédé pour mettre en oeuvre une exploitation combinée d'une installation de production de bioéthanol et d'une installation de biogaz,
**caractérisé en ce que**
- les drêches produites dans l'installation de production de bioéthanol sont introduites dans une installation de biogaz pour transformer les substances organiques contenues en biogaz, et
- il est prévu un retour direct d'une partie de l'effluent de l'installation de biogaz à des fins de macération dans le fermenteur de l'installation de production de bioéthanol.

2. Procédé selon la revendication 1,
**caractérisé en ce que**
l'effluent de l'installation de biogaz est soumis, avant le retour direct, partiellement à une étape de séparation de solides.

3. Procédé selon la revendication 2,
**caractérisé en ce que**
l'étape de séparation comprend une filtration et/ou une décantation.

4. Procédé selon l'une des revendications précédentes,
**caractérisé en ce que**
un flux partiel est enlevé par éclusage hors de l'effluent de l'installation de biogaz, qui est purifié au moyen d'un procédé à membrane, et de plus le perméat obtenu parvient complètement ou partiellement dans l'installation de production de bioéthanol pour la macération.

5. Procédé selon l'une des revendications précédentes,
**caractérisé en ce que**
un flux partiel est enlevé par éclusage hors de l'effluent de l'installation de biogaz, il est évaporé et donc concentré, et le condensat parvient au moins partiellement dans l'installation de production de bioéthanol pour la macération.

6. Procédé selon l'une des revendications précédentes,
**caractérisé en ce que**
l'ammoniac formé dans l'installation de biogaz est enlevé par stripage jusqu'à une concentration inoffensive pour le processus dans son ensemble.
